# EUROPÄISCHE PATENTSCHRIFT

(11) **EP 2 671 611 B1**
(45) Veröffentlichungstag und Bekanntmachung des Hinweises auf die Patenterteilung: **10.01.2018**
(21) Anmeldenummer: 13167897.1
(22) Anmeldetag: 15.05.2013
(51) Int. Cl.: H02G 3/06, F16B 21/18

(54) **Verbinder und medizinisches Gerät zur extrakorporalen Blutbehandlung mit einem solchen Verbinder**
Connector and medical device with extracorporeal blood treatment with such a connector
Connecteur et appareil médical de traitement extracorporel du sang doté d'un tel connecteur

(30) Priorität: 06.06.2012 DE 102012104931
(43) Veröffentlichungstag der Anmeldung: 11.12.2013
(73) Patentinhaber: B. Braun Avitum AG, 34212 Melsungen (DE)
(72) Erfinder: Stenzel, Bruno, 26209 Hatten (DE); Schade, Andreas, 36199 Rotenburg (DE)
(74) Vertreter: Winter, Brandl, Fürniss, Hübner, Röss, Kaiser, Polte - Partnerschaft mbB

(56) Entgegenhaltungen:
- WO-A1-92/08060
- DE-A1- 19 614 684
- US-A- 1 849 604
- US-A- 4 610 468
- US-A- 4 640 534
- US-A- 4 844 409
- US-A1- 2002 185 327

## Beschreibung

Die Erfindung betrifft einen Verbinder zur lösbaren Anbringung in einer Öffnung des Gehäuses eines medizinischen Geräts, mit einem Verbindungselement, an welches ein Einmalartikel außerhalb des Gehäuses anschließbar ist.

Die Erfindung betrifft ferner ein medizinisches Geräts zur extrakorporalen Blutbehandlung mit einem solchen Verbinder.

Ein Verbinder gemäß dem Obergriff des Anspruchs 1 ist beispielsweise aus der US 2002/0185327 A1 bekannt. Weitere Verbinder sind beispielsweise aus US 4,460,534 A und US 4,610,468 A bekannt.

US 2002/0185327 A1 offenbart einen Verbinder, welcher über als Schnellverbinderclips ausgebildete Rückhaltestrukturen an einer Gehäusewand anbringbar ist. Die Rückhaltestrukturen sind im montierten Zustand des Verbinders in an dem Verbinder vorgesehenen Nuten aufgenommen und eingespannt. Zwischen den Rückhaltestrukturen und dem Gehäuse liegt Spiel vor bzw. der Verbinder ist im an dem Gehäuse montierten Zustand zwischen den beiden Rückhaltestrukturen axial bewegbar.

US 4,460,534 A beschreibt einen Verbinder zwischen einer Flüssigkeitsleitung und einem Gewindeanschluss an einer Struktur, wobei der flüssigkeitsleitungsseitige Anschluss des Verbinders an ein männliches Element und der gewindeanschlussseitige Anschluss des Verbinders an ein weibliches Element angekoppelt ist. Beide Elemente sind miteinander mithilfe eines Sprengrings verbunden. Das weibliche Element ist derart umfassend ausgeformt, dass es in der Lage ist, den gesamten Verbinder zu stützen.

In US 4,610,468 A wird ebenfalls ein Verbinder beschrieben, der ein männliches und ein weibliches Element aufweist, wobei das weibliche Element so massiv ausgeformt ist, dass es im Stande ist, die Stützung des Verbinders zu übernehmen. Die Verbindung zwischen den beiden Elementen wird durch einen Haltering mit einschwenkbaren Vorsprüngen bewerkstelligt.

DE 196 14 684 A1 beschreibt eine Vorrichtung zur unlösbaren und dichten Verbindung von Rohren beziehungsweise Rohrstutzen, WO 92/08060 A1 eine mögliche Ausführungsform eines Sprengrings.

In medizintechnischen Geräten besteht oftmals die Notwendigkeit, Schnittstellen zu Einmalartikeln herzustellen. Diese Schnittstellen können beispielsweise nötig werden, um eine Druck- und/oder Temperaturmessung und Überwachung im Einmalartikel oder den Durchfluss für Flüssigkeiten oder Gase zu ermöglichen. Auch die Einbindung von optisch, akustisch und/oder mechanisch wirkenden Messsystemen in Kreisläufe kann durch Schnittstellen ermöglicht werden, oder Filter und weitere Volumen können in ein Schlauchsystem eingebunden werden. Um eine Standardisierung in der Gestaltung dieser Schnittstellen zu erreichen, wird in vielen Fällen eine Verbindung mittels des genormten Luer-Konus hergestellt.

In medizinischen Geräten zur extrakorporalen Blutbehandlung (Dialyse) werden beispielsweise Einmalartikel eingesetzt, die wenigstens aus den zu- und abführenden Leitungen und mehreren Luftfängern ein sogenanntes Überleitsystem bilden, mit dem Blut des Patienten zu einem Dialysator und zum Patienten zurückgefördert wird. Derartige Überleitsysteme werden nach jeder Behandlung ausgetauscht und nicht wieder für andere Patienten verwendet.

Im Bereich der extrakorporalen Blutbehandlung ist es ferner Stand der Technik, zur Drucküberwachung des extrakorporalen Kreislaufes den Einmalartikel mit einem weiblichen Luer-Verbinder zu versehen, welcher sich mit dem am medizinischen Gerät befindlichen männlichen Gegenstück verbinden lässt. Diese Verbindung muss bei jeder Behandlung neu hergestellt werden, was bedeutet, dass der am medizinischen Gerät befindliche Teil der Verbindung verschleißen oder durch unsachgemäßen Gebrauch beschädigt werden kann. In einem solchen Fall muss ein Austausch des am medizinischen Gerät befindlichen Verbindungsteils möglich sein.

Der am medizinischen Gerät befindliche Teil kann aus verschiedenen Materialien hergestellt sein. Beispielsweise ist es bekannt, nichtrostende Edelstähle, Stähle mit Oberflächenbeschichtung zum Korrosionsschutz (z.B. verchromt oder vernickelt), Nichtedelmetalle mit Oberflächenbeschichtung (z.B. Eloxierung) oder Kunststoffe einzusetzen. Ferner kann die Befestigung der Verbinder im Gehäuse des medizinischen Geräts auf verschiedene Arten erfolgen, wobei dies insbesondere durch Verkleben oder Verschrauben erfolgen kann. Dabei muss die gewählte Befestigung in Umfangsrichtung wirkenden Kräfte aufnehmen, wie sie beim Anschrauben des Einmalartikels auftreten. Ferner muss die Befestigung axial wirkende Kräfte beispielsweise durch Zugkräfte am Einmalartikel aufnehmen können. Insgesamt sollte die Befestigung aber auch lösbar sein, um den Verbinder bei Bedarf austauschen zu können.

Eine kostengünstige Lösung zu Herstellung eines Verbinders ist die Fertigung als werkzeugfallendes Bauteil in Kunststoffspritzgusstechnik. Denkbare Werkstoffvarianten sind hierbei thermoplastische Kunststoffe wie z.B. POM, PA, PP, PEEK, PPSU, PSU oder PPS. Die Eigenschaften eines so hergestellten Verbinders erschweren jedoch die Anwendung der derzeitig als Stand der Technik bekannten Befestigungsmethoden oder stehen in Widerspruch mit der Anforderung der Austauschbarkeit.

Aufgabe der Erfindung ist es daher, einen Verbinder bereitzustellen, der die genannten Grundanforderungen erfüllt und dabei einfach herzustellen und zu montieren ist. Aufgabe der Erfindung ist es ferner, ein medizinisches Gerät mit einem solchen Verbinder als Schnittstelle bereitzustellen.

Erfindungsgemäß wird diese Aufgabe durch einen Verbinder gemäß dem unabhängigen Anspruch 1 gelöst. Vorteilhafte Weiterbildungen des Verbinders ergeben sich aus den Unteransprüchen 2-7. Die Aufgabe wird ferner durch ein medizinisches Gerät zur extrakorporalen Blutbehandlung gemäß Anspruch 8 gelöst. Vorteilhafte Weiterbildungen des Gerätes ergeben sich aus Unteranspruch 9. Der erfindungsgemäße Verbinder dient zur Kopplung eines Einmalartikels am Gehäuse eines medizinischen Geräts und umfasst ein Verbindungselement, an welches der Einmalartikel außerhalb des Gehäuses anschließbar ist. Erfindungsgemäß ist das Verbindungselement über einen hohlen Steg mit dem Anschluss verbunden. Der Anschluss ist durch die Öffnung des Gehäuses in das Gehäuseinnere einführbar. Der Verbinder umfasst ferner ein Federelement, das lösbar so an diesem Steg anbringbar ist, dass der Verbinder über das Federelement am Gehäuse axial federnd abgestützt ist..

Dabei kann eine Abstützung des Federelements am Gehäuse eine Anbringung auf der Innenseite einer Gehäusewand und/oder eine Anbringung des Federelements innerhalb der Gehäusewand oder entsprechend ausgeformter Verdickungen der Gehäusewand umfassen. Auch zusätzliche Bauteile, die an einer Gehäusewand angebracht werden, um das Federelement zu halten, zu fixieren und/oder im Bezug zum Verbinder zu spannen, sind als Teil das Gehäuses anzusehen. Das erfindungsgemäße Federelement erfüllt somit zum einen die Funktion eines Sicherungsrings und zum anderen die Funktion einer Biegefeder.

Durch das anbringbare Federelement kann der Verbinder axial am Gehäuse fixiert werden, wobei beispielsweise durch ein Federelement in Form einer Tellerfeder Fertigungstoleranzen und Änderungen der Einspannlänge kompensiert werden können. Ferner kann der Verbinder axial durch Zugkräfte belastet werden, ohne dass der Verbinder beschädigt wird, wenn beispielsweise an dem daran angebrachten Einmalartikel gezogen wird.

Der Verbinder kann ferner, insbesondere am Steg, Mittel zur Drehfixierung des Verbinders (10;10') innerhalb der Öffnung des Gehäuses aufweisen.

Die Mittel zur Drehfixierung oder radialen Verrastung des Verbinders innerhalb der Öffnung des Gehäuses stellen einen Verdrehschutz im Umfangsrichtung dar und können ferner dazu genutzt werden, den Verbinder gezielt am Gehäuse auszurichten. Dies kann beispielsweise für die Anbringung von anderen Systemen am Anschluss vorteilhaft sein.

In einer vorteilhaften Ausgestaltung kann das Federelement nicht nur zur axialen Festlegung des Verbinders innerhalb der Gehäuseöffnung, sondern bei entsprechender Ausgestaltung auch gleichzeitig als Mittel zur Drehsicherung verwendet werden.

Das Federelement kann auf den Steg axial oder radial aufschiebbar sein und am Steg kann zur axialen, formschlüssigen Fixierung des Federelements wenigstens eine Nut vorgesehen sein.

Das Federelement kann teller- oder blattfederförmig ausgebildet sein, eine zentrale Öffnung aufweisen und mit einer segmentförmigen Aussparung oder einen radialen Schlitz, der bis zur zentralen Öffnung (55) reicht, versehen sein, so dass das Federelement zum Aufstecken in die Nut (42) im Steg (40) auf- bzw. auseinandergespreizt werden kann. Dieser Vorgang erfolgt bei der Montage des Verbinders am Gehäuse, aber das Federelement kann auch auf einfache Weise wieder aus der Nut herausgezogen werden, wonach der Verbinder wieder aus dem Gehäuse gelöst werden kann. Hierdurch ist eine schnelle und werkzeuglose Montage und Demontage möglich, wobei keine Trocknungszeiten beispielsweise durch die sonst übliche Verklebung von Bauteilen berücksichtigt werden müssen. Ferner sind die Bauteile des Verbinders relativ unempfindlich gegenüber einer unsachgemäßen Handhabung.

Der Steg kann einen runden oder eckigen Außenquerschnitt haben, wobei ein eckiger Außenquerschnitt bereits für eine Drehfixierung des Stegs innerhalb der Öffnung des Gehäuses genutzt werden kann, wenn die Öffnung ebenfalls entsprechend ausgeformt ist. Die Mittel zur Drehfixierung des Verbinders innerhalb der Öffnung im Gehäuse können jedoch auch speziell ausgeformte Verrastungsgeometrien innerhalb der Öffnung und am Steg umfassen. Beispielsweise ist am Steg eine Nase und innerhalb der Öffnung im Gehäuse eine Nut vorgesehen, in welche die Nase axial eingeschoben wird.

In einem Ausführungsbeispiel der Erfindung sind der Anschluss, das Verbindungselement und der Steg als einteiliges Kunststoffspritzgussteil ausgeformt, was die Herstellung des Verbinders kostengünstig macht. Auch das Federelement kann so kostengünstig aus Kunststoff hergestellt werden. Denkbare Werkstoffvarianten sind hierbei bewährte thermoplastische Kunststoffe wie z.B. POM, PA, PP, PEEK, PPSU, PSU oder PPS. Dabei ermöglicht die Gestaltung des Verbinders ein kunststoffgerechtes Design.

Darüber hinaus kann der Verbinder durch Zugabe von Farbpartikeln farblich codiert sein, was ebenfalls kostengünstig durchzuführen ist.

Von der Erfindung umfasst ist ferner ein medizinisches Gerät zur extrakorporalen Blutbehandlung, umfassend ein Gehäuse, wobei das Gehäuse wenigstens eine Öffnung aufweist, in welche ein erfindungsgemäßer Verbinder eingebracht ist. Ein solches medizinisches Gerät stellt hierdurch eine standardisierte Schnittstelle zum Anschluss eines Einmalartikels wie einem Umleitsystem bereit. An den Anschluss innerhalb des Gehäuses können jegliche Systeme angeschlossen werden, wobei es sich insbesondere um ein Manometer handeln kann.

Wird das medizinische Gerät dabei so ausgeführt, dass das Federelement von außerhalb des Gehäuses zugänglich ist, kann der Verbinder auch ohne Öffnen des Geräts getauscht werden.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:
- Fig. 1a: eine schematische Seitenansicht eines ersten Ausführungsbeispiels eines Verbinders vor der Montage eines Federelements;
- Fig. 1b: einen Verbinder nach Fig. 1b mit einem von innen montierten Federelement;
- Fig. 2: eine schematische Seitenansicht eines Verbinders;
- Fig. 3: eine schematische Aufsicht auf ein Federelement;
- Fig. 4: eine schematische Seitenansicht eines zweiten Ausführungsbeispiels eines fixierten Verbinders mit einem von außen montierten Federelement; und
- Fig. 5: eine Untersicht auf einen montierten Verbinder nach Fig. 4.

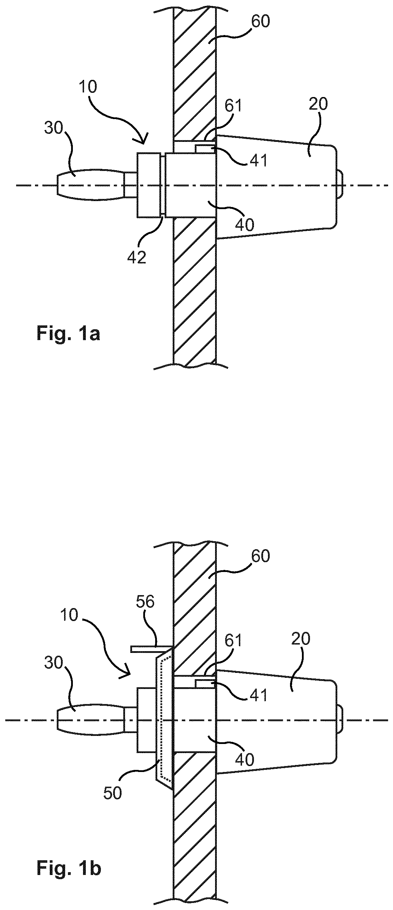

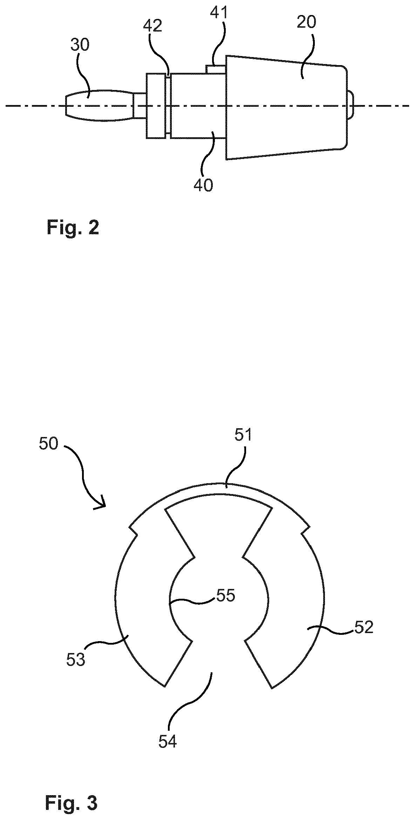

Fig. 1a zeigt ein erstes Ausführungsbeispiel eines erfindungsgemäßen Verbinders 10, der bereits innerhalb einer Öffnung in einem Gehäuse bzw. einer Gehäusewand 60 eines medizinischen Geräts eingebracht ist, aber noch nicht durch ein Federelement fixiert wurde. Fig. 1b zeigt den gleichen Verbinder 10 mit montiertem Federelement 50. Fig. 2 zeigt dagegen den Verbinder 10 losgelöst von dem Gehäuse 60.

Der Verbinder 10 weist ein Verbindungselement 20 und einen Anschluss 30 auf, die durch einen hohlen Steg 40 miteinander verbunden ist. Vorzugsweise handelt es sich bei dem Verbindungselement 20 um einen männlichen Luer-Konus, auf den ein Einmalartikel in Form eines Schlauchs aufgeschoben werden kann. Der Anschluss 30 kann ebenfalls als derartiger Schlauchanschluss ausgebildet sein. Der Verbinder 10 dient zur Kopplung eines (nicht gezeigten) Einmalartikels außerhalb des Gehäuses 60 des medizinischen Geräts mit einer (nicht gezeigten) Einrichtung, z.B: Messeinrichtung, innerhalb des Gehäuses 60.

Der Anschluss 30 ist dabei innerhalb des Gehäuses 60 positioniert, während das Verbindungselement 20 außerhalb des Gehäuses 60 angebracht ist. Der verbindende Steg 40 durchdringt das Gehäuse 60 in einer Öffnung, wobei ein Abschnitt des Stegs 40 in den Innenraum des Gehäuses 60 ragt. An diesem Abschnitt ist wenigstens eine Nut 42 vorgesehen, wie sie auch in Fig. 2 zu sehen ist. Die Nut 42 kann komplett oder abschnittsweise umlaufend ausgebildet sein, z.B. befinden sich wenigstens zwei gegenüber liegende Nuten an dem Steg. In diese Nut 42 ist dann in der Darstellung der Fig. 1b ein Federelement 50 eingeschoben. Das Federelement 50 ist tellerförmig so ausgeformt, dass es zwischen der Nut 42 und der Innenseite des Gehäuses 60 eingespannt werden kann. Diese Tellerform ist in Fig. 1b gestrichelt dargestellt, und das Verbindungselement 20 liegt von außen an dem Gehäuse 60 an und ist durch die Federkraft der Federelements 50 gegen die Außenwand des Gehäuses 60 vorgespannt. Über den Steg 40 ist der Verbinder 10 radial in der Gehäuseöffnung abgestützt.

Ferner ist am Steg 40 wenigstens eine Nase 41 bzw. eine Feder vorgesehen, welche innerhalb der Öffnung im Gehäuse 60 in eine entsprechende axial verlaufende Nut 61 eingreift, wodurch der Steg 40 und damit der Verbinder 10 innerhalb des Gehäuses 60 in Umfangsrichtung fixiert sind.

Ein Ausführungsbeispiel eines Federelements 50 ist in Fig. 3 in einer Aufsicht gezeigt. Das Federelement 50 ist insgesamt wie eine Tellerfeder ausgebildet. Der Außenrand des Federelements 50 ist, wie in der Fig. 1b gezeigt, schräg angestellt und bewirkt die federelastische Verformung des Federelements 50. Ferner weist das Federelement 50 in der Mitte eine Öffnung 55 auf. Neben dieser Öffnung 55 sind zwei Seitenteile 52 und 53 angeordnet und über einen Verbindungsbogen 51 miteinander verbunden. Gegenüber dem Verbindungsbogen 51 ist eine segmentförmige Aussparung 54 vorgesehen, welche bis zur Öffnung 55 hin ragt. Der Verbindungsbogen 51 ist elastisch ausgebildet, so dass die beiden Seitenteile 52 und 53 gegen eine Federkraft des Verbindungsbogens 51 auseinander gespreizt werden können.

Für eine Montage des Verbinders 10 am Gehäuse 60 werden der Anschluss 30 und der Steg 40 durch die Öffnung in das Gehäuse 60 geschoben, so dass das äußere Verbindungselement 20 am Gehäuse 60 anliegt und sich die Nut 42 am Steg 40 innerhalb des Gehäuses 60 befindet. Anschließend wird das Federelement 50 seitlich, beispielsweise von oben in die Nut 42 im Steg 40 eingebracht, indem die Aussparung 54 auf den Steg 40 gedrückt wird. Die Flanken der Aussparung 54 öffnen sich radial nach außen, so dass sich das Federelement 50 beim Aufschieben auf den Steg 40 auseinander gespreeizt wird. Die beiden Seitenteile 52 und 53 werden dabei auseinander gedrückt, und das Federelement 50 kommt mit den Innenkanten der Öffnung 55 in der Nut 42 zum Liegen. Wie bereits erwähnt, wird das Federelement 50 so eingeführt, dass durch die Tellerform eine Federkraft in axialer Richtung erzeugt wird.

Um den Verbinder 10 zu demontieren, kann das Federelement 50 wieder aus der Nut 42 herausgezogen werden. Dazu können kleine Griffflächen oder Ausbuchtungen vorgesehen sein, um das Federelement 50 gut greifen zu können. Im Ausführungsbeispiel der Fig. 1b ist dazu beispielsweise im oberen Bereich ein Griffelement 56 vorgesehen. Anstelle eines Griffelements kann auch eine Öse vorgesehen sein, in die ein Zugmittel eingreifen kann.

Bei dem Ausführungsbeispiel der Figuren 1a und 1b ist das Federelement 50 somit von innen montierbar, d.h. es muss ein Zugriff auf das Innere des Gehäuses 60 möglich sein, um den Verbinder 10 in einer Öffnung der Gehäusewand zu montieren oder demontieren zu können. Dazu kann die betreffende Gehäusewand beispielsweise entfernt werden, um an ihre Rückseite gelangen zu können.

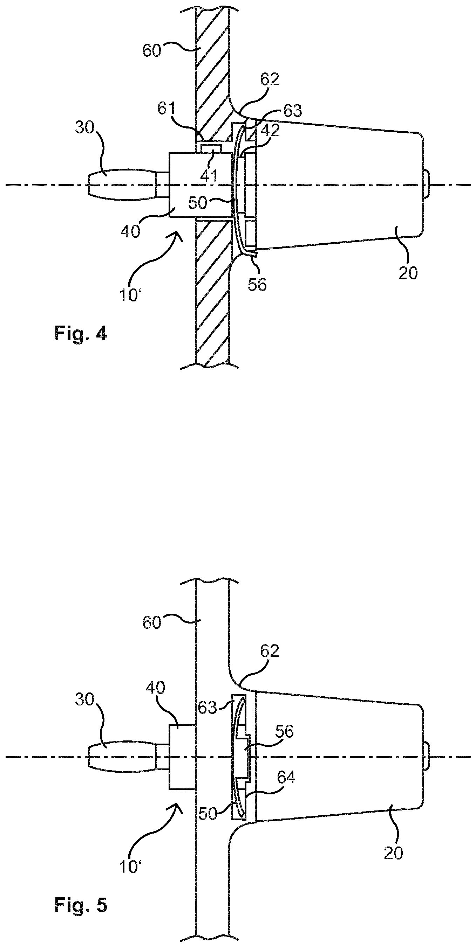

In einem zweiten Ausführungsbeispiel der Erfindung ist das Federelement 50 dagegen von außen montierbar. Dies kann auf unterschiedliche Arten realisiert werden, wobei Fig. 4 eine mögliche Ausführungsform eines solchen Verbinders 10' zeigt. Um das Federelement 50 von außen montieren und demontieren zu können, ist hierbei an der Außenseite des Gehäuses 60 wenigstens im Bereich des äußeren Verbindungselements 20 eine Ausbauchung oder Erhebung 62 ausgeformt, die auch als Abstützelement bezeichnet werden kann, an dem das Verbindungselement 20 anliegt und an dem es sich abstützt. Vorzugsweise handelt es sich um eine kreisrunde Erhebung, deren Umfang in etwa dem Umfang des Luer-Konus 20 entspricht. Die Erhebung 62 kann so ohne Stufe/Kante bündig in den Luer-Konus 20 übergehen.

Die Öffnung, in welche der Verbinder 10' eingebracht werden soll, erstreckt sich durch die Gehäusewand 60 und das Abstützelement 62 hindurch. Ferner ist innerhalb dieser Öffnung erneut eine Nut 61 vorgesehen, die zusammen mit einer Nase 41 am Steg 40 eine Drehsicherung des Verbinders 10 innerhalb des Gehäuses 60 gewährleistet. Die Nut 42 in dem Steg 40 ist nun jedoch im Bereich des Abstützelements 62 positioniert und ist vorzugsweise auch breiter ausgeführt als im ersten Ausführungsbeispiel des Verbinders 10, um das Einführen eines Federelements zu erleichtern.

Ferner befindet sich im Abstützelement 62 eine Tasche oder Aufnahme 63, in welche ein Federelement 50 seitlich, beispielsweise von unten eingeschoben werden kann. Das Federelement 50 kann erneut als Tellerfeder mit einer mittleren Öffnung und einer segmentförmigen Aussparung ausgeführt sein (siehe Fig. 3), so dass das Federelement 50 innerhalb der Nut 42 mit der Aussparung nach oben auf den Steg 40 geschoben werden kann, wobei sich die beiden Seitenteile des Federelements 50 auseinander spreizen. Um eine axiale Federspannung zu erzeugen, muss das tellerförmige Federelement 50 so in die Aufnahme 63 eingeführt werden, dass seine Außenränder an den rechten Innenflächen der Aufnahme 63 anliegen, der nach außen gebogene Bereich jedoch an der linken Innenfläche der Nut 42 im Steg 40 anliegt. Dazu muss das Federelement 50 beim Einführen leicht zusammengedrückt bzw. gebogen werden. Das Federelement 50 stützt sich dann an der Aufnahme 63 und dem Steg 40 ab, so dass es das Verbindungselement 20 durch seine Federkraft an das Abstützelement 62 heranzieht.

Dabei ist die Aufnahme 63 so an dem Abstützelement 62 angeordnet, dass das Federelement 50 von außen in die Aufnahme 63 eingeführt werden kann, ohne dass beispielsweise die Gehäusewand 60 demontiert werden muss. Dazu ist am Federelement 50 erneut ein Griffelement 56 oder Öse vorgesehen, mit dem das Federelement 50 von außen gegriffen werden kann.

Das Federelement 50 kann seitlich, beispielsweise von unten in die Aufnahme 63 eingeführt werden, wobei die Aufnahme 63 im Wesentlichen durch eine umlaufende Nut innerhalb des Abstützelements 62 und einen Öffnungsschlitz 64 im unteren Bereich des Abstützelements 62 ausgeformt ist. Dieser Öffnungsschlitz 64 ist schematisch der Untersicht der Fig. 5 zu entnehmen und erlaubt vorzugsweise ein Einführen des Federelements 50 zusammen mit dem Griffelement 56, wobei der Öffnungsschlitz 64 einen gewissen Spielraum ermöglichen sollte, denn das Federelement 50 muss unter Umständen etwas verdreht und gebogen werden, um es vorschriftsmäßig in die Aufnahme 63 und die Nut 42 im Steg 40 einführen zu können.

Der Öffnungsschlitz 64 kann auch seitlich oder oben am Abstützelement 62 angebracht sein, so dass das Federelement 50 entsprechend aus einer anderen Richtung auf den Steg 40 geschoben werden kann. Das Abstützelement 62 ist jedoch in jedem Fall als Teil des Gehäuses 60 anzusehen, so dass auch ein Anbringen des Federelements 50 innerhalb des Abstützelements 62 für diese Erfindung als Anbringung innerhalb des Gehäuses 60 anzusehen ist.

Ferner können optional Mittel zur Abdeckung des Öffnungsschlitzes 64 vorgesehen werden. Beispielsweise kann es sich hierbei um eine elastische Gummiabdeckung handeln, deren Geometrie an die Form des Öffnungsschlitzes 64 angepasst ist, so dass sie formschlüssig in den Öffnungsschlitz 64 gedrückt werden kann, aber auch leicht wieder entfernbar ist. Es kann jedoch beispielsweise auch vorgesehen sein, dass die Außenseite des Abstützelements 62 mit einem Außengewinde versehen ist, so dass ein Deckel mit einer mittleren Öffnung über den Luer-Konus 20 gestülpt und dann an dem Abstützelement 62 angeschraubt werden kann. Der Deckel könnte auch durch einzelne lösbare Clips- und/oder Schraubverbindungen am Gehäuse 60 fixiert werden, oder es handelt sich lediglich um einen elastischen Ring oder ein Gummiband, der/das so auf den Umfang des Abstützelements 62 gestülpt werden kann, dass er/es den Öffnungsschlitz 64 abdeckt.

Der Deckel würde dann den Öffnungsschlitz 64 abdecken und könnte wieder entfernt werden, um das Federelement 50 und damit den Verbinder 10' zu demontieren. Ein Deckel mit Schraubverbindung(en) zum Abstützelement könnte unter Umständen auch dazu genutzt werden, die erforderliche Federspannung im Federelement 50 zu erzeugen oder zu verstärken. In einem solchen Ausführungsbeispiel könnte das Federelement 50 leichter in eine Aufnahme innerhalb des Abstützelements 62 eingebracht werden, da die erforderliche Federspannung für eine axiale Sicherung erst durch Aufschrauben des Deckels erzeugt würde, indem der Deckel direkt oder indirekt Druck auf das Federelement ausübt. Der Deckel wäre in diesem Fall ebenfalls als Teil des Gehäuses anzusehen, wobei an der Schraubverbindung jedoch Verschleißerscheinungen auftreten könnten, so dass der Nutzen eines solches Deckels im Einzelfall gegen seine Nachteile abgewogen werden muss. Darüber hinaus könnte der Öffnungsschlitz 64 an der Unterseite des Stegs 40 auch ohne zusätzliche Abdeckung ausreichend gegen das Eindringen von Flüssigkeiten/Schmutz und einen unbefugten Zugriff geschützt sein.

### Bezugszeichenliste

- 10,10': Verbinder
- 20: Verbindungselement, Luer-Konus
- 30: Anschluss
- 40: Steg
- 41: Nase, Feder
- 42: Nut an Verbinder
- 50: Federelement
- 51: Verbindungsbogen
- 52,53: Seitenteil
- 54: Aussparung
- 55: Öffnung
- 56: Griffelement
- 60: Gehäuse, Gehäusewand
- 61: Nut in Gehäuse
- 62: Abstützelement
- 63: Aufnahme
- 64: Öffnungsschlitz

## Patentansprüche

1. Verbinder (10; 10'), der in einer Öffnung eines Gehäuses (60) eines medizinischen Geräts lösbar anbringbar ist und ein Verbindungselement (20), insbesondere in der Form eines männlichen Luer-Konus, aufweist, an welches ein Einmalartikel außerhalb des Gehäuses (60) anschließbar ist, wobei das Verbindungselement (20) über einen hohlen Steg (40) mit einem Anschluss (30) verbunden ist, der durch die Öffnung des Gehäuses (60) in das Gehäuseinnere einführbar ist, und der Verbinder (10; 10') ferner ein teller- oder blattfederförmiges Federelement (50) umfasst, welches eine zentrale Öffnung (55) aufweist und zum Aufstecken in eine am Steg (40) vorgesehene Nut (42) aufspreizbar ist, wobei das Federelement (50) eine segmentförmige Aussparung (54) oder einen Schlitz, der bis zur zentralen Öffnung (55) reicht, aufweist und lösbar am Steg (40) anbringbar ist, **dadurch gekennzeichnet, dass** der Verbinder (10; 10') über das Federelement (50) am Gehäuse (60) axial federnd abgestützt ist und dass im am Gehäuse (60) montierten Zustand des Verbinders (10; 10') das Verbindungselement (20) von außen an dem Gehäuse (60) anliegt und mittels des Federelements (50) gegen die Außenwand des Gehäuses (60) vorgespannt ist.

2. Verbinder (10; 10') nach Anspruch 1, **dadurch gekennzeichnet, dass** am Verbinder (10; 10'), insbesondere am Steg (40), Mittel zur Drehfixierung des Verbinders (10; 10') innerhalb der Öffnung des Gehäuses (60) vorgesehen sind.

3. Verbinder (10; 10') nach Anspruch 1 oder 2, **dadurch gekennzeichnet, dass** das Federelement (50) auf den Steg (40) axial oder radial aufschiebbar ist, wobei am Steg (40) zur axialen, formschlüssigen Fixierung des Federelements (50) wenigstens eine Nut (42) vorgesehen ist.

4. Verbinder (10; 10') nach einem oder mehreren der Ansprüche 1 bis 3, **dadurch gekennzeichnet, dass** die Mittel zur radialen Fixierung des Verbinders (10; 10') mit einer innerhalb der Öffnung im Gehäuse (60) komplementär ausgebildeten Verrastungsgeometrie zusammenwirken.

5. Verbinder (10; 10') nach Anspruch **4,dadurch gekennzeichnet, dass** die Drehfixierung über eine Feder-Nut-Verbindung erfolgt, insbesondere über eine am Steg (40) ausgebildete Feder (41), die in eine in der Öffnung des Gehäuses (60) ausgebildete axial verlaufende Nut (61) einführbar ist.

6. Verbinder (10; 10') nach einem oder mehreren der Ansprüche 1 bis 5, **dadurch gekennzeichnet, dass** der Anschluss (30), das Verbindungselement (20) und der Steg (40) als einteiliges Kunststoffspritzgussteil ausgeformt sind.

7. Verbinder (10; 10') nach einem oder mehreren der Ansprüche 1 bis 6, **dadurch gekennzeichnet, dass** der Verbinder (10; 10') durch Zugabe von Farbpartikeln farblich codiert ist.

8. Medizinisches Gerät zur extrakorporalen Blutbehandlung mit einem Gehäuse (60), **dadurch gekennzeichnet, dass** das Gehäuse (60) wenigstens eine Öffnung aufweist, in welche ein Verbinder (10; 10') gemäß einem der Ansprüche 1 bis 7 eingebracht ist.

9. Medizinisches Gerät nach Anspruch 8, **dadurch gekennzeichnet, dass** das Federelement (50) von außerhalb des Gehäuses (60) zugänglich ist.

## Claims

1. Connector (10; 10') which can be releasably fitted in an opening of a housing (60) of a medical device and which has a connection element (20), in particular in the form of a male Luer cone, to which a disposable article can be connected outside the housing (60), wherein the connection element (20) is connected by means of a hollow web (40) to a connection (30) which can be introduced through the opening of the housing (60) into the inside of the housing, and the connector (10; 10') further comprises a disc-spring-like or leaf-spring-like resilient element (50) which has a central opening (55) and which can be expanded for insertion into a groove (42) which is provided on the web (40), wherein the resilient element (50) has a segment-like recess (54) or a slot which extends as far as the central opening (55), and which can be releasably fitted to the web (40), **characterised in that** the connector (10; 10') is supported in an axially resilient manner on the housing (60) by means of the resilient element (50) and **in that**, in the state of the connector (10; 10') mounted on the housing (60), the connection element (20) is in abutment with the housing (60) from outside and is pretensioned by means of the resilient element (50) against the outer wall of the housing (60).

2. Connector (10; 10') according to claim 1, **characterised in that** on the connector (10; 10'), in particular on the web (40), means for rotationally fixing the connector (10; 10') are provided inside the opening of the housing (60).

3. Connector (10; 10') according to claim 1 or 2, **characterised in that** the resilient element (50) can be pushed axially or radially onto the web (40), wherein at least one groove (42) is provided on the web (40) for axial positive-locking fixing of the resilient element (50).

4. Connector (10; 10') according to any one of claims 1 to 3, **characterised in that** the means for radially fixing the connector (10; 10') cooperate with an engagement geometry which is constructed in a complementary manner inside the opening in the housing (60).

5. Connector (10; 10') according to claim 4, **characterised in that** the rotational fixing is carried out by means of a tongue and groove connection, in particular by means of a tongue (41) which is formed on the web (40) and which can be introduced in an axially extending groove (61) which is formed in the opening of the housing (60).

6. Connector (10; 10') according to one or more of claims 1 to 5, **characterised in that** the connection (30), the connection element (20) and the web (40) are formed as a one-piece injection-moulded plastics material component.

7. Connector (10; 10') according to one or more of claims 1 to 6, **characterised in that** the connector (10; 10') is colour-coded by means of the addition of colour particles.

8. Medical device for extracorporeal blood treatment having a housing (60), **characterised in that** the housing (60) has at least one opening in which a connector (10; 10') according to any one of claims 1 to 7 is introduced.

9. Medical device according to claim 8, **characterised in that** the resilient element (50) is accessible from outside of the housing (60).

## Revendications

1. Raccord (10; 10'), qui peut être appliqué de manière amovible dans une ouverture d'un boîtier (60) d'un appareil médical et présente un élément de jonction (20), en particulier sous la forme d'un cône Luer mâle, auquel peut être raccordé un article à usage unique en dehors du boîtier (60), dans lequel l'élément de jonction (20) est relié via une barrette creuse (40) à un branchement (30) qui peut être inséré à travers l'ouverture du boîtier (60) à l'intérieur du boîtier et le raccord (10; 10') comprend en outre un élément élastique (50) sous la forme d'une rondelle-ressort ou d'un ressort à lames qui présente une ouverture centrale (55) et peut s'écarter lors de l'enfichage dans une rainure (42) ménagée dans la barrette (40), dans lequel l'élément élastique (50) présente un évidement en forme de segment (54) ou une fente qui s'étend jusqu'à l'ouverture centrale (55) et peut s'appliquer de manière amovible sur la barrette (40), **caractérisé en ce que** le raccord (10; 10') s'appuie axialement de manière élastique sur le boîtier (60) via l'élément élastique (50) et, à l'état du raccord (10; 10') monté sur le boîtier (60), l'élément de jonction (20) s'applique de l'extérieur sur le boîtier (60) et est précontraint contre la paroi externe du boîtier (60) au moyen de l'élément élastique (50).

2. Raccord (10; 10') selon la revendication 1, **caractérisé en ce que,** sur le raccord (10; 10'), en particulier sur la barrette (40), il est prévu des moyens pour fixer par rotation le raccord (10; 10') à l'intérieur de l'ouverture du boîtier (60).

3. Raccord (10; 10') selon la revendication 1 ou 2, **caractérisé en ce que** l'élément élastique (50) peut coulisser axialement ou radialement sur la barrette (40), dans lequel il est prévu sur la barrette (40) au moins une rainure (42) pour la fixation axiale de l'élément élastique (50) avec adaptation de formes.

4. Raccord (10; 10') selon une ou plusieurs des revendications 1 à 3, **caractérisé en ce que** les moyens de fixation radiale du raccord (10; 10') coopèrent avec une géométrie d'enclenchement conformée de manière complémentaire à l'intérieur de l'ouverture dans le boîtier (60).

5. Raccord (10; 10') selon la revendication 4, **caractérisé en ce que** la fixation par rotation se fait via une jonction ressort-rainure, en particulier via un ressort (41) formé sur la barrette (40), lequel ressort peut être inséré dans une rainure (61) s'étendant axialement ménagée dans l'ouverture du boîtier (60).

6. Raccord (10; 10') selon une ou plusieurs des revendications 1 à 5, **caractérisé en ce que** le branchement (30), l'élément de jonction (20) et la barrette (40) sont formés par une pièce de moulage par injection en matière plastique d'un seul tenant.

7. Raccord (10; 10') selon une ou plusieurs des revendications 1 à 6, **caractérisé en ce que** le raccord (10; 10') est codé en couleur par addition de particules de colorant.

8. Appareil médical pour le traitement extracorporel du sang avec un boîtier (60), **caractérisé en ce que** le boîtier (60) présente au moins une ouverture dans laquelle un raccord (10; 10') selon l'une quelconque des revendications 1 à 7 est introduit.

9. Appareil médical selon la revendication 8, **caractérisé en ce que** l'élément élastique (50) est accessible de l'extérieur du boîtier (60).
